# EUROPEAN PATENT SPECIFICATION

(11) **EP 3 010 882 B1**
(45) Date of publication and mention of the grant of the patent: **04.04.2018**
(21) Application number: 14740044.4
(22) Date of filing: 20.06.2014
(51) Int. Cl.: C07C 253/34, C07C 255/04, C07C 255/07

(54) **EXTRACTION SOLVENT CONTROL FOR REDUCING STABLE EMULSIONS**
STEUERUNG EINES EXTRAKTIONSLÖSUNGSMITTELS ZUR REDUZIERUNG STABILER EMULSIONEN
CONTRÔLE DE SOLVANT D'EXTRACTION POUR LA RÉDUCTION D'ÉMULSIONS STABLES

(30) Priority: 20.06.2013 US 201361837477 P
(43) Date of publication of application: 27.04.2016
(73) Proprietor: INVISTA Textiles (U.K.) Limited, Manchester M2 3DE (GB)
(72) Inventor: TENN, William, J., III, Beaumont, TX 77706 (US)
(74) Representative: Carpmaels & Ransford LLP
(86) International application number: PCT/US2014/043394
(87) International publication number: WO 2014/205337

(56) References cited:
- WO-A1-2013/095853
- DE-A1-102004 045 036

## Description

### FIELD OF THE INVENTION

The invention relates to recovery of catalyst and ligand from a hydrocyanation reaction product mixture comprising organic dinitriles using liquid-liquid extraction.

### BACKGROUND OF THE INVENTION

It is well known in the art that complexes of nickel with phosphorous-containing ligands are useful as catalysts in hydrocyanation reactions. Such nickel complexes using monodentate phosphites are known to catalyze hydrocyanation of butadiene to produce a mixture of pentenenitriles. These catalysts are also useful in the subsequent hydrocyanation of pentenenitriles to produce adiponitrile, an important intermediate in the production of nylon. It is further known that bidentate phosphite, phosphinite and phosphonite ligands can be used to form nickel-based catalysts to perform such hydrocyanation reactions.

U.S. Patent No. 3,773,809 describes a process for the recovery of Ni complexes of organic phosphites from a product fluid containing organic nitriles produced by hydrocyanating an ethylenically unsaturated organic mononitrile such as 3-pentenenitrile through extraction of the product fluid with a paraffin or cycloparaffin hydrocarbon solvent. Similarly, U.S. Patent No. 6,936,171 to Jackson and McKinney discloses a process for recovering diphosphite-containing compounds from streams containing dinitriles.

U.S. Patent No. 4,339,395 describes the formation of an interfacial rag layer during extended periods of continuous extraction of certain phosphite ligands. The '395 patent notes that the interfacial rag hinders, if not halts, the phase separation. Because the process is operated continuously, the rag must be removed continuously from the interface as it accumulates to avoid interrupting operation. To solve this problem for the disclosed components, the '395 patent discloses the addition of minor amounts of substantially water-free ammonia.

U.S. Patent No. 7,935,229 describes a process for extractively removing heterogeneously dissolved catalyst from a reaction effluent of a hydrocycanation of unsaturated mononitriles to dinitriles with a hydrocarbon. The catalyst comprises a ligand which may be a monophosphite, a diphosphite, a monophosphonite or a diphosphonite. Ammonia or an amine may be added to a mixture of liquid phases before phase separation takes place.

International Patent Publication No. WO 2013/095853, describes a process corresponding to that described in the present application, except that a diphosphite ligand is recovered in the process of International Patent Publication No. WO 2013/095853, whereas a diphosphonite ligand is recovered in the process of the present application.

### SUMMARY OF THE INVENTION

This process recovers diphosphonite-containing compounds from a mixture comprising diphosphonite-containing compounds, organic mononitriles and organic dinitriles.

Disclosed is a process for recovering diphosphonite-containing compounds from a feed mixture comprising diphosphonite-containing compounds, organic mononitriles, organic dinitriles and a Lewis acid in a multistage countercurrent liquid-liquid extractor with extraction solvent comprising aliphatic hydrocarbon, cycloaliphatic hydrocarbon or a mixture of aliphatic and cycloaliphatic hydrocarbon. The process comprises:
a) flowing the feed mixture to the first stage of the multistage countercurrent liquid-liquid extractor; and
b) contacting the feed mixture with extraction solvent from the light phase of the second stage of the multistage countercurrent liquid-liquid extractor;
wherein the first stage of the multistage countercurrent liquid-liquid extractor comprises a mixing section and a settling section, wherein a light phase separates from a heavy phase in the settling section, wherein a Lewis base is added to the mixing section of the first stage of the multistage countercurrent liquid-liquid extractor, wherein the light phase comprises extraction solvent and extracted diphosphonite-containing compounds, wherein the heavy phase comprises organic mononitriles, organic dinitriles and a complex of said Lewis acid and said Lewis base, wherein at least a portion of the light phase is withdrawn from the settling section and treated to recover diphosphonite-containing compounds extracted into the light phase, and wherein at least a portion of the heavy phase is passed to the second stage of the multistage countercurrent liquid-liquid extractor, wherein the Lewis base is selected from the group consisting of:
a) anhydrous ammonia, pyridine, alkylamine, dialkylamine, trialkylamine wherein the alkyl groups have 1 to 12 carbon atoms; and
b) polyamine.

The mixing sections of the stages of the multistage counter current liquid-liquid extractor form an intimate mixture of unseparated light and heavy phase. This intimate mixture comprises an emulsion phase. The emulsion phase may or may not comprise particulate solid material. This emulsion phase separates into a light phase and a heavy phase in the settling sections of the stages, including the first stage. Accordingly, the settling sections of the stages will contain at least some emulsion phase located between the upper light phase and the lower heavy phase. This emulsion phase tends to reduce in size over time. However, in some instances settling takes longer than desired or the emulsion phase never fully separates into a light phase and a heavy phase. This separation problem may be particularly troublesome in the first stage of a multistage countercurrent liquid-liquid extractor.

Addition of Lewis base to the mixing section of the first stage has been found to result in enhanced settling of the emulsion phase. For example, this addition may result in the reduction of the size of the emulsion phase in the settling section, wherein the size of the emulsion phase is based upon the size of the emulsion phase in the absence of addition of Lewis base. Enhanced settling in the settling section may also be measured as an increased rate of settling, based upon the rate of settling in the absence of addition of Lewis base.

Another problem, which may be solved by addition of Lewis base, is formation of rag and build-up of a rag layer the settling section. Rag formation is discussed in U.S. Patent No. 4,339,395 and U.S. Patent No. 7,935,229. Rag comprises particulate solid material, and may be considered to be a form of an emulsion phase, which is particularly stable in the sense that it does not dissipate in a practical amount of time for conducting an extraction process. Rag may form in the mixing section or the settling section of an extraction stage, particularly the first stage of a multistage countercurrent liquid-liquid extractor. In the settling section, the rag forms a layer between the heavy phase and the light phase. The formation of a rag layer in the settling section inhibits proper settling of the heavy phase and the light phase. The formation of a rag layer may also inhibit the extraction of diphosphonite-containing compounds from the heavy phase into the light phase. In a worst case scenario, rag can build up to the extent of completely filling a separation section, necessitating shut down of the extraction process to clean out the settling section. It has been found that addition of Lewis base to the mixing section may reduce or eliminate the size of a rag layer or reduce its rate of formation, based upon the size and rate of formation of the rag layer in the absence of addition of Lewis base.

Accordingly, addition of Lewis base to the mixing section of the first stage of a multistage countercurrent extractor may achieve at least one of the following results: (a) a reduction in the size of an emulsion phase in the settling section, based upon the size of the emulsion phase in the absence of addition of Lewis base; (b) an increase in the rate of settling in the settling section, based upon the rate of settling in the absence of addition of Lewis base; (c) an increase in the amount of diphosphonite-containing compounds in the light phase, based upon the upon the amount of diphosphonite-containing compounds in the light phase in the absence of addition of Lewis base; (d) a partial or total reduction in the size of a rag layer in the settling section, based upon the size of a rag layer in the settling section in the absence of addition of Lewis base; and (e) reduction in the rate of formation of a rag layer in the settling section, based upon the rate of formation of a rag layer in the settling section in the absence of addition of Lewis base.

In one embodiment, Lewis base is cofed to the mixing section along with the feed mixture comprising diphosphonite-containing compounds, organic mononitriles, organic dinitriles and Lewis acid. In another embodiment, Lewis base is separately fed to the mixing section apart from the feed mixture comprising diphosphonite-containing compounds, organic mononitriles, organic dinitriles and Lewis acid and the extraction solvent feed.

A particular example of a Lewis acid, which may be present in the feed to the extractor, is ZnCl₂.

The extraction solvent feed from the second stage may comprise at least 1000 ppm, for example, from 2000 to 5000 ppm, of diphosphonite-containing compounds. The extraction solvent feed from the second stage may comprise at least 10 ppm, for example, from 20 to 200 ppm, of nickel.

The diphosphonite-containing compound may be a diphosphonite ligand of formula I:

(R¹)(R²-O)P-O-Y-O-P(O-R³)(R⁴) I

where R¹ and R² are each independently identical or different, separate or bridged organic radicals; R³ and R⁴ are each independently identical or different, separate or bridged organic radicals; and Y is a bridging group.

Examples of phosphonite-containing compounds of formula (I) may be diphosphonite ligands of formula (II) or (formula III): 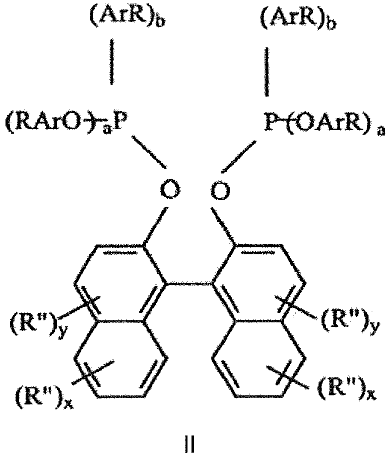 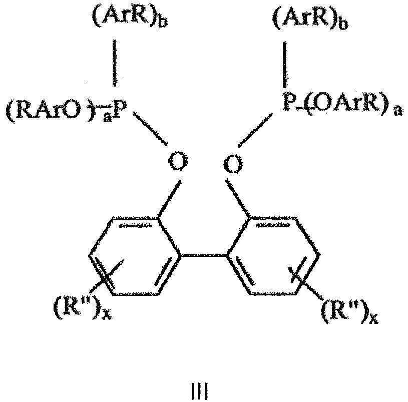 wherein:
x=0 to 4;
y=0 to 2;
a is 1 and b is 1;
each Ar is individually phenyl or naphthyl, and the two Ar groups that are directly or indirectly (through an oxygen) bonded to the same phosphorus atom may be linked to each other by a linking unit selected from the group consisting of direct bond, alkylidene, secondary or tertiary amine, oxygen, sulfide, sulfone, and sulfoxide;
each R is individually hydrogen, ethenyl, propenyl, acryloyl, methacryloyl, an organic radical with a terminal ethenyl, propenyl, acryloyl, or methacryloyl group, linear or branched alkyl, cycloalkyl, acetal, ketal, aryl, alkoxy, cycloal koxy, aryloxy, formyl, ester, fluorine, chlorine, bromine, perhaloalkyl, hydrocarbylsulfinyl, hydrocarbylsulfonyl, hydrocarbylcarbonyl or cyclic ether;
each Ar can be further substituted with linear or branched alkyl, cycloalkyl, acetal, ketal, aryl, alkoxy, cycloalkoxy, aryloxy, formyl, ester, fluorine, chlorine, bromine, perhaloalkyl, hydrocarbylsulfinyl, hydrocarbylsulfonyl, hydrocarbylcarbonyl or cyclic ether;
each R" is individually hydrogen, ethenyl, propenyl, an organic radical with a terminal ethenyl or propenyl group, linear or branched alkyl, cycloalkyl, acetal, ketal, aryl, alkoxy, cycloalkoxy, aryloxy, formyl, ester, fluorine, chlorine, bromine, perhaloalkyl, hydrocarbylsulfinyl, hydrocarbylsulfonyl, hydrocarbylcarbonyl or cyclic ether.

Examples of diphosphonite ligands of formula (III) include compounds where at least one R represents ethenyl, propenyl, acryloyl, methacryloyl or the organic radical with a terminal ethenyl, propenyl, acryloyl, or methacryloyl group or at least one R" represents ethenyl, propenyl, or the organic radical with a terminal ethenyl or propenyl group.

An example of a diphosphonite ligand of formula (III) is a compound of formula (IV):

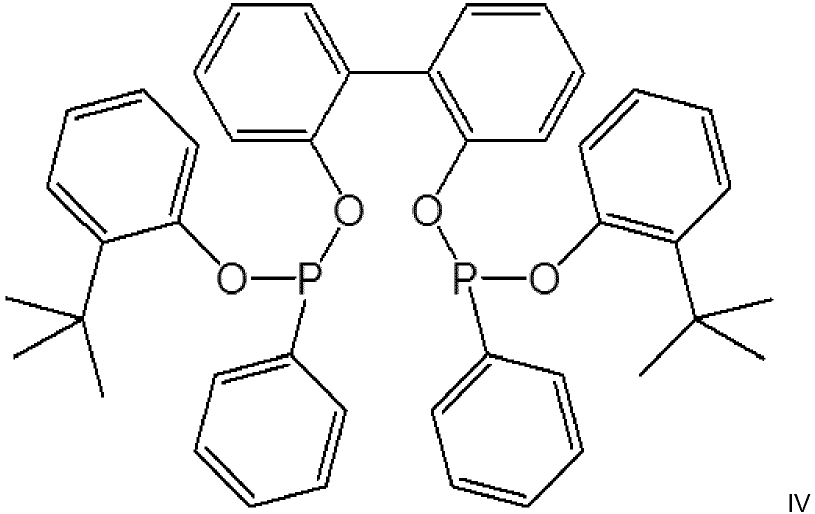

Diphosphonite ligands and the synthesis of these diphosphonite ligands are described in U.S. Patent No. 6,924,345 and in U.S. Patent No. 7,935,229.

At least a portion of the diphosphonite ligand may be complexed with zero valent Ni.

At least one stage of the extraction may be carried out above 40°C.

The Lewis base may be a monodentate triarylphosphite or a monodentate triarylphosphine, wherein the aryl groups are unsubstituted or substituted with alkyl groups having 1 to 12 carbon atoms, and wherein the aryl groups may be interconnected.

The Lewis base used in the invention is selected from the group consisting of:
a) anhydrous ammonia, pyridine, alkylamine, dialkylamine, trialkylamine wherein the alkyl groups have 1 to 12 carbon atoms; and
b) polyamine.

If the Lewis base is a polyamine, the polyamine may comprise at least one selected from hexamethylene diamine, and dimers and trimers of hexamethylene diamine, for example, bis-hexamethylene triamine.

The Lewis base may optionally comprise a basic ion exchange resin, for example, Amberlyst 21® resin.

One example of a suitable cyclic alkane extraction solvent is cyclohexane.

At least a portion of the process may be carried out in an extraction column or a mixer-settler.

The feed mixture may be an effluent stream from a hydrocyanation process, for example, a process for hydrocyanating 3-pentenenitrile, a process for the single hydrocyanation of 1,3-butadiene to pentenenitriles or a process for the double hydrocyanation of 1,3-butadiene to adiponitrile.

The first stage of the multistage countercurrent liquid-liquid extractor may take place in an extraction column. The entire column may be considered to be a settling section comprising a mixing section between a heavy phase collection section and a light phase collection section. Heavy phase may be recycled to the mixing section of the extraction column.

The first stage of the multistage countercurrent liquid-liquid extractor may take place in a mixer-settler. The mixer-settler may comprise a settling section which is separate from the mixing section. Recycled heavy stream may be recycled upstream from the point of withdraw of the recycled heavy stream.

### BRIEF DESCRIPTION OF THE DRAWINGS

Figure 1 is a diagram showing the flow of fluids through a multistage countercurrent liquid-liquid extractor.
Figure 2 is a diagram showing a mixing section and a settling section of a stage of a multistage countercurrent liquid-liquid extractor.
Figure 3 is a diagram showing an extraction column.
Figure 4 is a diagram showing a mixing/settling apparatus having three chambers in the settling section.
Figure 5 is a diagram showing a distillation train which may be used to recover adiponitrile from a raffinate stream.
Figures 6 and 7 are graphs showing the conversion of adiponitrile to 2-cyanocyclopentylideneimine (CPI) in the presence of various additives over time.

### DETAILED DESCRIPTION OF THE INVENTION

The processes of the present invention involve methods for recovering diphosphonite-containing compounds from a mixture comprising diphosphonite-containing compounds and organic dinitriles, using liquid-liquid extraction.

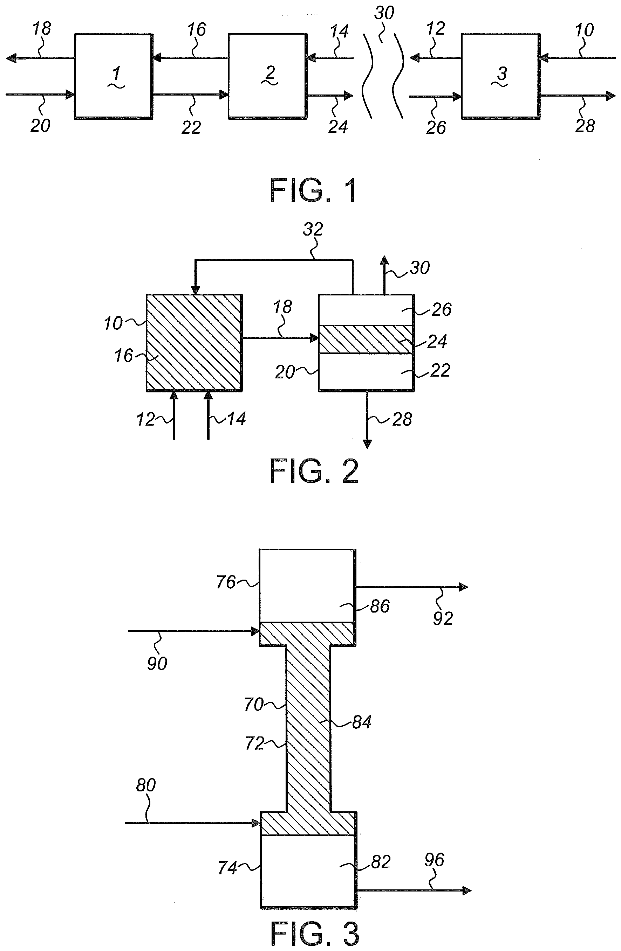

Figure 1 is a diagram of a multistage countercurrent liquid-liquid extractor. Lines in Figure 1 represent flow of materials, rather than any particular type of equipment, such as pipes. Similarly, squares in this diagram represent stages or sections for mixing and settling, rather than any particular type of equipment.

Three stages are depicted in Figure 1. The first stage is depicted by mixing and settling section **1.** The second stage is depicted by mixing and settling section **2.** The final stage is depicted by mixing and settling section **3.** Gap **30** represents a space where additional stages may be inserted. For example, one or more, for example, from one to four, mixing and settling sections may be inserted in gap **30** between mixing and settling section **2** and mixing and settling section **3.**

In Figure 1, a fresh extraction solvent feed, for example, cyclohexane, is introduced into the multistage countercurrent extractor via line **10.** The extraction solvent or light phase exiting from mixing settling section **3** passes through line **12** to the next stage of the multistage extractor. In a multistage countercurrent liquid-liquid extractor having three stages, extraction solvent in line **12** would pass directly into stage **2** via line **14.** Extraction solvent from stage **2** passes through line **16** to stage **1.** The extraction solvent comprising extracted diphosphonite-containing compounds passes out of the stage **1** mixing and settling section through line **18.**

A feed comprising diphosphonite-containing compounds is fed into the stage 1 mixer and settler via line **20.** The feed further comprises a mixture comprising organic mononitriles and dinitriles, which is immiscible with the extraction solvent. The feed further comprises a Lewis acid. In stage **1,** a portion of the diphosphonite-containing compounds is extracted into the extraction solvent which exits stage **1** via line **18.** The immiscible dinitrile and mononitrile mixture or the heavy phase is removed from the stage **1** mixing and settling section by line **22** and is passed into the stage **2** mixing and settling section. A portion of the diphosphonite-containing compounds is extracted into the light phase in the stage **2** mixing and settling section. The heavy phase exits the stage **2** mixing and settling section by line **24.** Similarly, if there are additional stages in gap **30** shown in Figure 1, extraction of diphosphonite-containing compounds will take place in such intermediate stages in a similar manner to that taking place in stage **2.**

After the heavy phase passes through the first stage and any intermediate stages, it passes through the final stage mixing and settling section **3.** In particular, the heavy phase is introduced into mixing and setting section **3** through line **26.** After passing through the final stage mixing and settling section **3,** the heavy phase exits via line **28.**

A two-stage multistage countercurrent liquid-liquid extractor is represented in Figure 1 by mixing and settling sections **1** and **2;** lines **14, 16** and **18** showing the direction of extraction solvent flow; and lines **20, 22** and **24** showing the direction of heavy phase flow. In a two-stage multistage counter current liquid-liquid extractor, mixing and settling section **3;** lines **10, 12, 26** and **28;** and gap **30** are omitted. In the two stage countercurrent liquid-liquid extractor, extraction solvent comprising extracted diphosphonite-containing compounds passes from the extractor through line **18,** and extracted heavy phase, i.e. raffinate, passes from the extractor through line **24.**

Thus, it can be seen that the multistage countercurrent liquid-liquid extractor comprises two or more stages with countercurrent flow of extraction solvent and heavy phase. In view of the direction of flow of light and heavy phase through the stages of extraction, it will be appreciated that the concentration of solute, e.g., diphosphonite-containing compound, is highest in both the light and heavy phases of the first stage and lowest in the light and heavy phases of the final stage.

Figure 2 is a diagrammatic representation of one type of a mixing and settling section, also referred to herein as a mixer-settler. This type of mixer-settler may be used in any of the stages shown in Figure 1. This mixer-settler comprises a mixing section **40** and a settling section **50.** The mixing section **40** and the settling section **50** are separate. All of the effluent from the mixing section **40** flows into the settling section **50.** Fluid from the mixing section **40** flows through the settling section **50** in a horizontal manner, although there is also no restriction of movement of fluids vertically throughout the settling section **50.**

An extraction solvent is introduced into the mixing section **40** by line **42.** A feed comprising diphosphonite-containing compounds is introduced into the mixing section **40** by line **44.** Alternatively, the contents of lines **42** and **44** may be combined upstream of the mixing section **40** and introduced into mixing section **40** through a single inlet. These two feeds are mixed in the mixing section **40** to provide a mixed phase comprising an emulsion phase represented in Figure 2 by shaded area **46.**

Line **48** represents the flow of mixed phase **46** from the mixing section **40** into the settling section **50.** As depicted in Figure 2, there are three phases in the settling section **50,** including a heavy phase **52,** a mixed phase **54,** and a light phase **56.** The heavy phase **52** is depleted in diphosphonite-containing compounds, insofar as it has a lower concentration of diphosphonite-containing compounds as compared with the concentration of diphosphonite-containing compounds in feed **44,** due to the extraction of diphosphonite-containing compounds into the light phase **56.** Correspondingly, the light phase **56** is enriched in diphosphonite-containing compounds, insofar as it has a higher concentration of diphosphonite-containing compounds as compared with the concentration of diphosphonite-containing compounds in extraction solvent feed **42,** due to the extraction of diphosphonite-containing compounds into the light phase **56.** At least a portion of the heavy phase **52** exits the settling section **50** via line **60.** At least a portion of the light phase **56** is removed from the settling section **50** via line **58.**

Although not shown in Figure 2, which is diagrammatically shows the flow of fluids, it will be understood that each of the mixing section **40** and the settling section **50** may comprise one or more stages, subsections, compartments or chambers. For example, settling section **50** may include more than one chamber between the point of introduction of the mixed phase **46** through line **48** and the point of withdrawal of light phase and heavy phase through lines **58** and **60.** Horizontal extension between the point of introduction of the mixed phase **46** through line **48** and the point of withdrawal of light and heavy phases through lines **58** and **60** promotes settling of the light and heavy phases **56** and **52.** The size of the mixed phase **54** may become progressively smaller as fluids settle and flow through the chamber. For example, the final chamber from where fluids are removed may include little or no mixed phase **54.** It will further be understood that mixing section **40** may include one or more types of mixing apparatus, such as an impeller, not shown in Figure 2.

Figure 3 provides a representation of another type of apparatus for use as a mixing and settling section. The type of apparatus **70** shown in Figure 3 is referred to herein as an extraction column. This extraction column **70** includes a mixing section **72,** a heavy phase collection section **74** and a light phase collection section **76.** The entire column **70** may be considered to be a settling section with a mixing section between collection section **74** and collection section **76.** In extraction column **70** the mixing section **72** is part of the settling section. An extraction solvent is introduced into column **70** through line **80.** A heavier phase comprising a diphosphonite-containing compound is introduced into column **70** through line **90.** As the light phase passes upward through the column, and the heavy phase passes downward through the column, a mixture of the two phases is formed in mixing section **72.** This mixture is represented in Figure 3 as shaded mixed phase **84.** This mixed phase **84** may comprise an emulsion phase. The point of introduction of heavy phase through line **90** should be sufficiently above the point of introduction of the light phase to allow for sufficient mixing of the two phases in the mixing section resulting in the extraction of diphosphonite-containing compounds into the light phase. The intimate mixing of light and heavy phase in mixing section **72** may be promoted by mechanical or static mixing apparatus not shown in Figure 3. For example, mixing section **72** may comprise baffles or perforated plates, not shown in Figure 3.

The heavy phase **82** settles into collection section **74** and passes out of the column **70** through line **96.** Light phase **86** settles in collection section **76** and passes from the column through line **92.**

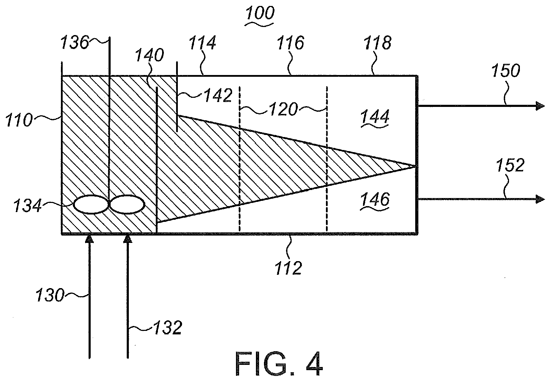

Figure 4 provides a representation of a mixer-settler **100** having as multistage settling section. Mixer-settler **100** has a mixing section **110** and a settling section **112**. In mixer-settler **100,** the mixing section **110** is separate from the settling section **112.** The settling section has three compartments, represented in Figure 4 as sections **114, 116,** and **118.** These sections are separated by coalescence plates **120.** The coalescence plates **120** may be designed to provide flow of separated light and heavy phases between chambers, while restricting the flow of emulsion phase between chambers. A feed comprising a diphosphonite-containing compound is passed into the mixing section **110** via line **130.** The extraction solvent is introduced into mixing section **110** via line **132.** The mixing section **110** includes an impeller **134** mounted on shaft **136** to provide for mechanical mixing of fluids. Mixing of the feeds provides a mixed phase comprising an emulsion phase represented in Figure 4 by shading **140.**

The mixed phase **140** flows into the settling section **112** as an overflow from the mixing section **110.** This mixed phase **140** is prevented from flowing directly into the light phase **144** by baffle plate **142.** As settling occurs in settling section **112,** the volume of the mixed phase **140** decreases, the volume of the light phase **144** increases, and the volume of the heavy phase **146** increases. Heavy phase **146** is removed from settling section **112,** in particular from chamber **118,** via line **152** and light phase **144** is removed from settling section **112,** in particular, from chamber **118,** via line **150.**

It is desirable for both a mononitrile and a dinitrile to be present in the countercurrent contactor. For a discussion of the role of monodentate and bidentate ligand in extraction of hydrocyanation reactor effluent streams, see U.S. Patent No. 3,773,809 to Walter and U.S. Patent 6,936,171 to Jackson and McKinney.

For the process disclosed herein, suitable molar ratios of mononitrile to dinitrile components include 0.01 to 2.5, for example, 0.01 to 1.5, for example 0.65 to 1.5.

Maximum temperature is limited by the volatility of the hydrocarbon solvent utilized, but recovery generally improves as the temperature is increased. Examples of suitable operating ranges are 40°C to 100°C and 50°C to 80°C.

The controlled addition of monophosphite ligands may enhance settling. Examples of monophosphite ligands that may be useful as additives include those disclosed in Drinkard et al U.S. Patent 3,496,215, U.S. Patent 3,496,217, U.S. Patent 3,496,218, U.S. Patent 5,543,536, and published PCT Application WO 01/36429 (BASF).

As described herein, the addition of Lewis base compounds to a mixture comprising diphosphonite-containing compounds, organic mononitriles and organic dinitriles enhances settling, especially when the mixture comprises a Lewis acid, such as ZnCl₂. Examples of suitable weak Lewis base compounds include water and alcohols. Suitable stronger Lewis base compounds used in the invention include hexamethylene diamine, dimers and trimers of hexamethylene diamine, ammonia, aryl- or alkyl amines, such as pyridine or triethylamine. Examples of other stronger Lewis based compounds include basic resins such as Amberlyst 21®, a commercially available basic resin made by Rohm and Haas. The addition of Lewis base may reduce or eliminate any inhibiting effect of Lewis acid on catalyst recovery.
The reaction product of Lewis acid with Lewis base may become entrained in the raffinate phase as it moves through the multistage countercurrent liquid-liquid extractor. In particular, this product may form a precipitate in the raffinate phase in the form of a complex of Lewis acid with Lewis base. However, this precipitate may exist as a dispersion of fine particles distributed throughout the raffinate phase. This precipitate may be removed by conventional techniques, such as filtration, centrifugation or distillation accompanied by removal of bottoms containing the precipitate, after the raffinate is removed from the last stage of the multistage countercurrent liquid-liquid extractor.

The diphosphonite-containing compounds extracted by the processes described herein comprise bidentate phosphorus-containing ligands. These extracted ligands comprise free ligands (e.g., those which are not complexed to a metal, such as nickel) and those which are complexed to a metal, such as nickel. Accordingly, it will be understood that extraction processes described herein are useful for recovering diphosphonite-containing compounds which are metal/ligand complexes, such as a complex of zero valent nickel with at least one ligand comprising a bidentate-phosphorus containing ligand.

### Diphosphonite Ligands

The diphosphonite-containing compound may be a diphosphonite ligand of formula (I):

(R¹)(R²-O)P-O-Y-O-P(O-R³)(R⁴) I

where R¹ and R² are each independently identical or different, separate or bridged organic radicals; R³ and R⁴ are each independently identical or different, separate or bridged organic radicals; and Y is a bridging group.

The R¹ and R² radicals may each independently be identical or different organic radicals. Examples of R¹ and R² radicals are aryl radicals, preferably those having from 6 to 10 carbon atoms, which may be unsubstituted or mono- or polysubstituted, in particular by C₁-C₄-alkyl, halogen, such as fluorine, chlorine, bromine, halogenated alkyl, such as trifluoromethyl, aryl, such as phenyl, or unsubstituted aryl groups.

The R³ and R⁴ radicals may each independently be identical or different organic radicals. Examples of R³ and R⁴ radicals are aryl radicals, preferably those having from 6 to 10 carbon atoms, which may be unsubstituted or mono- or polysubstituted, in particular by C₁-C₄-alkyl, halogen, such as fluorine, chlorine, bromine, halogenated alkyl, such as trifluoromethyl, aryl, such as phenyl, or unsubstituted aryl groups.

The R¹ and R² radicals may each be separate or bridged. The R³ and R⁴ radicals may also each be separate or bridged. The R¹, R², R³ and R⁴ radicals may each be separate, two may be bridged and two separate, or all four may be bridged.

Examples of phosphonite-containing compounds of formula (I) may be diphosphonite ligands of formula (II) or (formula III): 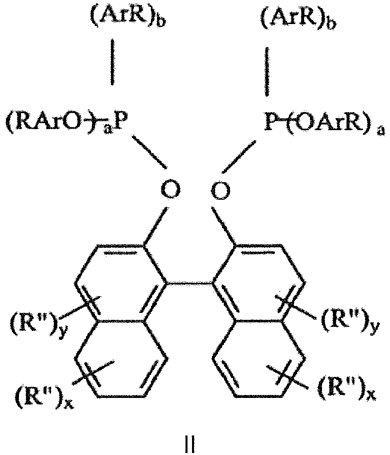 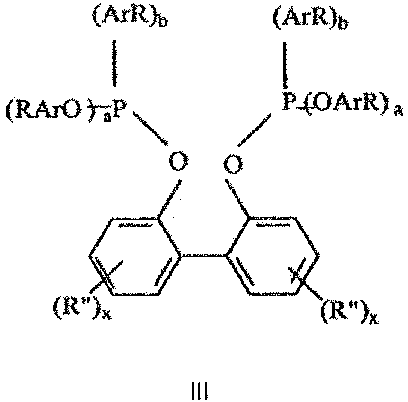 wherein:
x=0 to 4;
y=0 to 2;
a is 1 and b is 1;
each Ar is individually phenyl or naphthyl, and the two Ar groups that are directly or indirectly (through an oxygen) bonded to the same phosphorus atom may be linked to each other by a linking unit selected from the group consisting of direct bond, alkylidene, secondary or tertiary amine, oxygen, sulfide, sulfone, and sulfoxide;
each R is individually hydrogen, ethenyl, propenyl, acryloyl, methacryloyl, an organic radical with a terminal ethenyl, propenyl, acryloyl, or methacryloyl group, linear or branched alkyl, cycloalkyl, acetal, ketal, aryl, alkoxy, cycloal koxy, aryloxy, formyl, ester, fluorine, chlorine, bromine, perhaloalkyl, hydrocarbylsulfinyl, hydrocarbylsulfonyl, hydrocarbylcarbonyl or cyclic ether;
each Ar can be further substituted with linear or branched alkyl, cycloalkyl, acetal, ketal, aryl, alkoxy, cycloalkoxy, aryloxy, formyl, ester, fluorine, chlorine, bromine, perhaloalkyl, hydrocarbylsulfinyl, hydrocarbylsulfonyl, hydrocarbylcarbonyl or cyclic ether;
each R" is individually hydrogen, ethenyl, propenyl, an organic radical with a terminal ethenyl or propenyl group, linear or branched alkyl, cycloalkyl, acetal, ketal, aryl, alkoxy, cycloalkoxy, aryloxy, formyl, ester, fluorine, chlorine, bromine, perhaloalkyl, hydrocarbylsulfinyl, hydrocarbylsulfonyl, hydrocarbylcarbonyl or cyclic ether;

At least one R in formula (II) or formula (III) may represent ethenyl, propenyl, acryloyl, methacryloyl or the organic radical with a terminal ethenyl, propenyl, acryloyl, or methacryloyl group and/or at least one R" may represent ethenyl, propenyl, or the organic radical with a terminal ethenyl or propenyl group.

An example of a diphosphonite ligand of formula (III) is a compound of formula (IV):

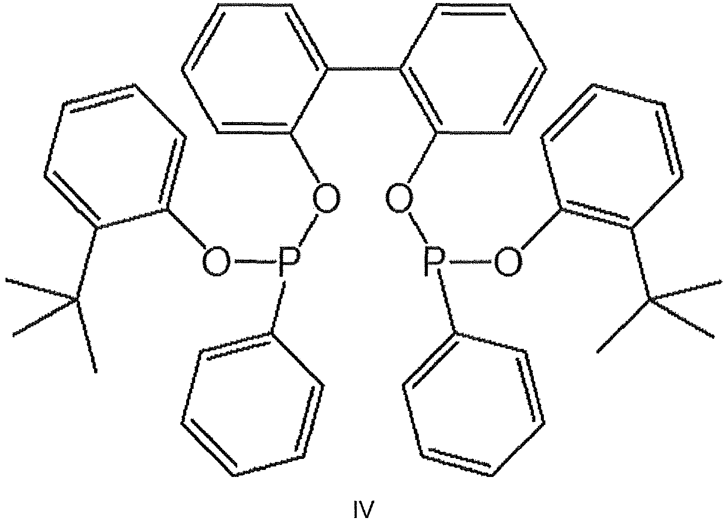

Diphosphonite ligands and the synthesis of these diphosphonite ligands are described in U.S. Patent No. 6,924,345 and in U.S. Patent No. 7,935,229.

### Extraction Solvent

Suitable hydrocarbon extraction solvents include paraffins and cycloparaffins (aliphatic and alicyclic hydrocarbons) having a boiling point in the range of about 30 °C to about 135 °C, including n-pentane, n-hexane, n-heptane and n-octane, as well as the corresponding branched chain paraffinic hydrocarbons having a boiling point within the range specified. Useful alicyclic hydrocarbons include cyclopentane, cyclohexane and cycloheptane, as well as alkyl substituted alicyclic hydrocarbons having a boiling point within the specified range. Mixtures of hydrocarbons may also be used, such as, for example, mixtures of the hydrocarbons noted above or commercial heptane which contains a number of hydrocarbons in addition to n-heptane. Cyclohexane is the preferred extraction solvent.

### Recovery of Products

The lighter (hydrocarbon) phase recovered from the multistage countercurrent liquid-liquid extractor is directed to suitable equipment to recover catalyst, reactants, etc. for recycle to the hydrocyanation, while the heavier (lower) phase containing dinitriles recovered from the multistage countercurrent liquid-liquid extractor is directed to product recovery after removal of any solids, which may accumulate in the heavier phase. These solids may contain valuable components which may also be recovered, e.g., by the process set forth in U.S. Patent No. 4,082,811.

The solids in the heavier phase, also referred to herein as the raffinate phase, comprise a complex of Lewis acid and Lewis base in the form of disperision of fine particles. The raffinate phase may also comprise extraction solvent, such as cyclohexane, pentenenitriles, which comprise 3-pentenenitrile, compounds with a higher boiling point than adiponitrile and compounds with a boiling point greater than the boiling point of pentenenitriles and less than the boiling point of adiponitrile. The complex of Lewis acid and Lewis base may be removed from the raffinate phase prior to removing extraction solvent, and especially before removing pentenenitriles from the raffinate phase.

The complex of Lewis acid and Lewis base may be removed by any customary solids removal process. Examples of such processes include filtration, crossflow filtration, centrifugation, sedimentation, classification and decantation. Common apparatus for such solids removal include filters, centrifuges and decanters.

It has been found that the complex of Lewis acid and Lewis base may catalyze the unwanted cyclization reaction of adiponitrile to form 2-cyanocyclopentylideneimine (CPI), especially when the raffinate phase is heated to temperatures used in the K'₄ column, discussed hereinafter, which is used to separate adiponitrile from other dinitriles, such as methylglutaronitrile and ethylsuccinonitrile.

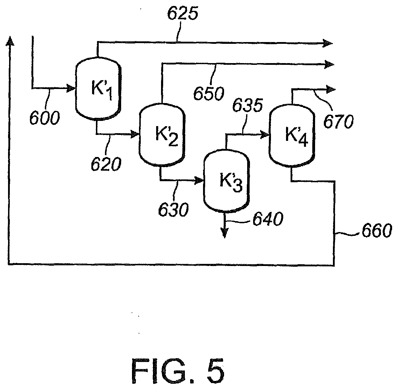

Figure 5 shows a distillation train, which may be used as an adiponitrile purification section. Figure 5 of the present application corresponds to Figure 3 of United States Patent Application Publication No. 2013/0211126. Line **600** transports a raffinate stream from an extraction zone into distillation column K'₁, where extraction solvent is separated from higher boiling components of the raffinate stream. In particular, extraction solvent, such as cyclohexane, is withdrawn from distillation column K'₁ through line **625,** and higher boiling components of the raffinate stream are withdrawn from distillation column K'₁ through line **620.**

The solvent-depleted stream in line **620** is then passed into distillation column K'₂, where pentenenitrile is separated from higher boiling components remaining in the raffinate stream. In particular, pentenenitrile, such as 3PN and any 2M3BN present, is withdrawn from distillation column K'₂ through line **650,** and higher boiling components of the raffinate stream are withdrawn from distillation column K'₂ through line **630.**

The pentenenitrile-depleted stream in line **630** is then passed into distillation column K'₃, where dinitriles are separated from higher boiling components remaining in the raffinate stream. In particular, dinitriles, such as ADN and MGN, are withdrawn from distillation column K'₃ through line **635,** and higher boiling components of the raffinate stream are withdrawn from distillation column K'₃ through line **640.** These higher boiling components in line **640** may comprise, for example, catalyst degradation products.

The dinitrile-enriched stream in line **635** is then passed into distillation column K'₄, where adiponitrile is separated from lower boiling dinitriles, such as MGN. In particular, MGN is withdrawn from distillation column K'₄ through line **670,** and a purified adiponitrile stream is withdrawn from distillation column K'₄ through line **660.**

Although not shown in Figure 5, a complex of Lewis acid and Lewis base in the form of a dispersed solid precipitate may be removed, e.g., by filtration, from the raffinate before the stream is introduced into distillation column K'₁. According to another embodiment, this complex may be removed from the stream in line **620** before this stream enters distillation column K'₂. According to another embodiment, this complex may be removed from the stream in line **630** before this stream enters distillation column K'₃.

### EXAMPLES

In the following examples, values for extraction coefficient are the ratio of weight fraction of catalyst in the extract phase (hydrocarbon phase) versus the weight fraction of catalyst in the raffinate phase (organonitrile phase). An increase in extraction coefficient results in greater efficiency in recovering catalyst. As used herein, the terms, light phase, extract phase and hydrocarbon phase, are synonymous. Also, as used herein, the terms, heavy phase, organonitrile phase and raffinate phase, are synonymous.

Analyses of the extract and the raffinate streams of the catalyst extraction were conducted on an Agilent 1100 series HPLC and via ICP. The HPLC was used to determine the extraction efficiency of the process.

In the Examples which follow, a diphosphite ligand is present. However, it is believed that the results of these Examples would be essentially the same if a diphosphonite ligand were substituted for the diphosphite ligand.

### Example 1

To a 50 mL, jacketed, glass laboratory extractor, equipped with a magnetic stirbar, digital stir-plate, and maintained at 65°C, was charged 10 grams of the product of a pentenenitrile-hydrocyanation reaction, and 10 grams of the extract from the second stage of a mixer-settler cascade, operated in counter-current flow. This extract from the second stage comprised approximately 50 ppm nickel and 3100 ppm diphosphite ligand. The hexamethylene diamine concentration in the system was 0 ppm.

The reactor product was approximately:
85% by weight C₆ dinitriles
14% by weight C₅ mononitriles
1% by weight catalyst components
200 ppm by weight active nickel
230 ppm by weight zinc.

The laboratory reactor was then mixed at 500 rotations-per-minute, for 10 minutes, and then allowed to settle for 1 minute. After settling for 1 minute, a stable emulsion was present throughout the extract phase. Samples were obtained of the extract and raffinate phases of the extractor and analyzed to determine the extent of catalyst extraction. The ratio of active nickel present in the extract phase vs. the raffinate phase was found to be 5. The concentration of zinc in the raffinate was found to be 230 ppm.

### Example 2

Example 1 was repeated except that hexamethylene diamine (HMD) was added to the system. In particular, a sufficient amount of HMD was added so that the molar ratio of Zn/HMD was 12 in the system.

### Example 3

Example 1 was repeated except that hexamethylene diamine (HMD) was added to the system. In particular, a sufficient amount of HMD was added so that the molar ratio of Zn/HMD was 6 in the system.

### Example 4

Example 1 was repeated except that hexamethylene diamine (HMD) was added to the system. In particular, a sufficient amount of HMD was added so that the molar ratio of Zn/HMD was 2.4 in the system.

### Example 5

Example 1 was repeated except that hexamethylene diamine (HMD) was added to the system. In particular, a sufficient amount of HMD was added so that the molar ratio of Zn/HMD was 1.2 in the system.

### Example 6

Example 1 was repeated except that bis-hexamethylene triamine (BHMT) was added to the system. In particular, a sufficient amount of BHMT was added so that the molar ratio of Zn/BMHT was 5.9 in the system.

### Example 7

Example 1 was repeated except that bis-hexamethylene triamine (BHMT) was added to the system. In particular, a sufficient amount of BHMT was added so that the molar ratio of Zn/BMHT was 2.9 in the system.

### Example 8

Example 1 was repeated except that bis-hexamethylene triamine (BHMT) was added to the system. In particular, a sufficient amount of BHMT was added so that the molar ratio of Zn/BMHT was 1.2 in the system.

### Example 9

Example 1 was repeated except that bis-hexamethylene triamine (BHMT) was added to the system. In particular, a sufficient amount of BHMT was added so that the molar ratio of Zn/BMHT was 12 in the system.

### Example 10

Example 1 was repeated except that 1,2-diaminocyclohexane (DCH) was added to the system. In particular, a sufficient amount of DCH was added so that the molar ratio of Zn/DCH was 1.6 in the system.

### Example 11

Example 1 was repeated except that 1,2-diaminocyclohexane (DCH) was added to the system. In particular, a sufficient amount of DCH was added so that the molar ratio of Zn/DCH was 2 in the system.

### Example 12

Example 1 was repeated except that 1,2-diaminocyclohexane (DCH) was added to the system. In particular, a sufficient amount of DCH was added so that the molar ratio of Zn/DCH was 4 in the system.

### Example 13

Example 1 was repeated except that 1,2-diaminocyclohexane (DCH) was added to the system. In particular, a sufficient amount of DCH was added so that the molar ratio of Zn/DCH was 8 in the system.

### Example 14

Example 1 was repeated except that triethylamine (TEA) was added to the system. In particular, a sufficient amount of TEA was added so that the molar ratio of Zn/TEA was 1 in the system.

### Example 15

Example 1 was repeated except that octylamine was added to the system. In particular, a sufficient amount of TEA was added so that the molar ratio of Zn/octylamine was 1.3 in the system.

### Comparative Example 16

Example 1 was repeated except that polyethyleneglycol (PEG-600) was added to the system. In particular, a sufficient amount of PEG-600 was added so that the molar ratio of Zn/PEG-600 was 1.5 in the system.

### Comparative Example 17

Example 1 was repeated except that adipamide was added to the system. In particular, a sufficient amount of adipamide was added so that the molar ratio of Zn/ adipamide was 2.3 in the system.

### Comparative Example 18

Example 1 was repeated except that triphenyl phosphine (Ph₃P) was added to the system. In particular, a sufficient amount of Ph₃P was added so that the molar ratio of Zn/Ph₃P was 1 in the system.

### Reference Example 19

Example 1 was repeated except that calcium hydroxide (Ca(OH)₂) was added to the system. In particular, a sufficient amount of Ca(OH)₂ was added so that the molar ratio of Zn/Ca(OH)₂ was 0.3 in the system.

Results of Examples 1-19 are summarized in Table 1.

**Table 1**

| Ex./CEx./REx. | Temp (°C) | Time (min) | Zn/Additive | Additive | KLL | Zn/Ni |
|---|---|---|---|---|---|---|
| 1 | 65 | 10 | | None | 5 | 1.15 |
| 2 | 65 | 10 | 12.0 | HMD | 13 | 1.09 |
| 3 | 65 | 10 | 6.0 | HMD | 13 | 1.11 |
| 4 | 65 | 10 | 2.4 | HMD | 23 | 0.43 |
| 5 | 65 | 10 | 1.2 | HMD | 84 | 0.12 |
| 6 | 65 | 10 | 5.9 | BHMT | 102 | 0.12 |
| 7 | 65 | 10 | 2.9 | BHMT | 80 | 0.17 |
| 8 | 65 | 10 | 1.2 | BHMT | 112 | 0.17 |
| 9 | 65 | 10 | 12.0 | BHMT | 18 | |
| 10 | 65 | 10 | 1.6 | DCH | 119 | 0.85 |
| 11 | 65 | 10 | 2 | DCH | 114 | |
| 12 | 65 | 10 | 4 | DCH | 27 | 1.03 |
| 13 | 65 | 10 | 8 | DCH | 8 | 1.05 |
| 14 | 65 | 10 | 1 | TEA | 20 | 0.94 |
| 15 | 65 | 10 | 1.3 | Octylamine | 63 | 0.96 |
| 16 | 65 | 10 | 1.5 | PEG-600 | 5 | 1.07 |
| 17 | 65 | 10 | 2.3 | Adipamide | 6 | |
| 18 | 65 | 10 | 1 | Ph₃P | 4 | 1.15 |
| 19 | 65 | 10 | 0.3 | Ca(OH)₂ | 14 | |

| | | | | | | |
|---|---|---|---|---|---|---|
| KLL = amount of catalyst in the extract / amount of catalyst in the raffinate; Zn/Additive = the molar ratio of the zinc-to-additive during extraction; Zn/Ni = the ratio of the total amount of zinc-to-nickel remaining in both phases after the extraction, as determined by inductively coupled plasma spectrometry (ICP). | | | | | | |

The data summarized in Table 1 represent evaluations of a number of materials as potential additives for improved catalyst extraction. Examples 1-5 show the beneficial effect of hexamethylene diamine (HMD) on catalyst extraction, as the HMD loading increases (represented by decreasing Zn/Additive ratio) the catalyst extraction efficiency (represented by KLL) increases. Examples 6-9 show the beneficial effect of bis-hexamethylene triamine (BHMT) on catalyst extraction. Examples 10-13 show the beneficial effect of triethylamine (TEA) on catalyst extraction. Example 15 shows the beneficial effect of adding octylamine on catalyst extraction. Reference Example 19 shows the beneficial effect of calcium hydroxide on catalyst extraction. By way of contrast, Comparative Examples 16-18 show little effect on catalyst extraction using PEG-600, adipamide, and triphenyl phosphine, respectively.

The results in Table 1 show that BHMT produced superior results. For example, as compared with HMD, at a Zn/Additive ratio of 1.2, BHMT produced a greater KLL value than HMD. As compared with DCH, BHMT produced a greater KLL value and a smaller Zn/Ni ratio, when used at a Zn/Additive ratio of 5.9, than DCH, when used at a Zn/Additive ratio of 4. As compared with octylamine, BHMT produced a greater KLL value and a smaller Zn/Ni ratio, when used at a Zn/Additive ratio of 1.2, than octylamine, when used at a Zn/Additive ratio of 1.3.

### Examples 20-25

These Examples 20-25 illustrate that effective catalyst recovery occurs for a mononitrile to dinitrile ratio greater than 0.65.

Five different mixtures comprised of a Ni diphosphite complex, with the diphosphite ligand shown in Structure XX (where R¹⁷ is isopropyl, R¹⁸ is H, and R¹⁹ is methyl), ZnCl₂ (equimolar with Ni) and differing in the ratio of mononitrile to dinitrile, were separately liquid-liquid batch extracted with an equal weight of cyane (i.e. cyclohexane). The molar ratio of organic mononitrile to organic dinitrile and the resulting extraction coefficients are shown in the Table 2 below. A compound may be effectively recovered if it has an extraction coefficient of 1 or greater at solvent to feed ratios greater than 1 using a countercurrent multistage extractor.

**Table 2.**

| **Catalyst and ligand extraction coefficients for varying ratios of mononitriles-to-dinitriles** | | | |
|---|---|---|---|
| Example | mononitrile/dinitrile | Catalyst extraction coefficient | Ligand extraction coefficient |
| 20 | 2.33 | 1.28 | 4.09 |
| 21 | 1.85 | 1.33 | 8.08 |
| 23 | 1.19 | 2.02 | 16.97 |
| 24 | 0.91 | 2.63 | 35.99 |
| 25 | 0.57 | 4.82 | 49.59 |

### Example 26

This Example demonstrates the effect of hold-up time on the extractability of the diphosphite ligand catalyst.

A mixture comprised predominantly of organic dinitriles and a Ni diphosphite complex, the structure of the diphosphite ligand being shown in Structure XX (where R¹⁷ is isopropyl, R¹⁸ is H, and R¹⁹ is methyl) and ZnCl₂ (equimolar with Ni) was divided into two portions. Both portions are liquid-liquid extracted in a three-stage contactor at 40°C, with an equal weight of cyclohexane. Both portions were sampled with time and the progress of the catalyst recovery into the extract phase is shown in Table 3 as the percent of the final steady state value achieved at a given time.

**Table 3**

| **Concentration of Diphosphite ligand with time in the extracting solvent phase.** | |
|---|---|
| Time, minutes | % of steady state concentration at 40°C |
| 2 | 12 |
| 4 | 19 |
| 8 | 34 |
| 14 | 52 |
| 30 | 78 |
| 60 | 100 |
| 91 | 100 |

### Example 27

This Example illustrates the effect of temperature on the extractability of catalyst with first-stage extraction solvent recycle.

A mixture comprised predominantly of organic dinitriles and a Ni diphosphite complex, the structure of the diphosphite ligand being shown in Structure XXIV (where R¹⁷ is methyl, R¹⁸ is methyl and R¹⁹ is H) and ZnCl₂ (equimolar with Ni) was divided into three portions. The portions were batch liquid-liquid extracted at 50°C, 65°C and 80°C, respectively, with an equal weight of n-octane and monitored with time. The results are shown in Table 4.

**Table 4**

| Time | % of steady state at 50°C | % of steady state at 65°C | % of steady state at 80°C |
|---|---|---|---|
| 2 | 0.0 | 0.0 | 1.8 |
| 4 | 0.0 | 0.0 | 1.6 |
| 8 | 0.0 | 0.0 | 3.6 |
| 14 | 0.0 | 0.0 | 4.3 |
| 20 | 0.0 | 0.0 | 3.6 |
| 30 | 0.0 | 0.0 | 7.6 |
| 60 | 0.0 | 1.6 | 16.3 |
| 90 | 0.7 | 4.0 | 48.6 |

### Example 28

This Example demonstrates the effect of adding water in three-stage extraction with cyclohexane recycle in the last stage.

Fifteen grams of a mixture comprised predominantly of organic dinitriles and a Ni diphosphite complex, the structure of the diphosphite ligand being shown in Structure XXIV (where R¹⁷ is methyl, R¹⁸ is methyl and R¹⁹ is H) and ZnCl₂ (equimolar with Ni), was extracted in a three-stage continuous extractor at a temperature of 50°C with an equal weight of cyclohexane for one hour resulting in an catalyst extraction coefficient of 4.3, as measured by the amount of catalyst in the extract of the first stage divided by the amount of catalyst in the feed of the reaction mixture fed to the last stage of the three-stage countercurrent extractor.

To this mixture, 100 microliters of water was added. After continuing to heat and agitate for another hour, the diphosphite Ni extraction coefficient was measured as 13.4 - a threefold increase.

### Examples 29 and 30

These Examples demonstrate the effect of adding hexamethylene diamine (HMD) to the extraction zone.

Example 1 was repeated except that hexamethylene diamine was added to the product of a pentene-hydrocyanation reaction. To a 50 mL, jacketed, glass laboratory extractor, equipped with a magnetic stirbar, digital stir-plate, and maintained at 65°C, was charged 10 grams of the product of pentene-hydrocyanation reactor product, and 10 grams of the extract from the second stage of a mixer-settler cascade, operated in counter-current flow.

The reactor product was approximately:
85% by weight C₆ dinitriles
14% by weight C₅ mononitriles
1% by weight catalyst components
360 ppm by weight active nickel.

The laboratory reactor was then mixed at 1160 rotations-per-minute, for 20 minutes, and then allowed to settle for 15 minutes. A stable emulsion was present throughout the extract phase in the absence of the addition of HMD. After 15 minutes of settling, essentially no emulsion phase was present when HMD was added. Samples were obtained of the extract and raffinate phases of the extractor and analyzed to determine the extent of catalyst extraction.

**Table 5**

| **Effect of hexamethylene diamine on catalyst extraction** | | | |
|---|---|---|---|
| Example | Concentration of HMD added (ppm) | Catalyst recovery (KLL) | Stable emulsion |
| 1 | 0 | 14 | Yes |
| 29 | 250 | 43 | No |
| 30 | 500 | 80 | No |

### Examples 31-36

These Examples demonstrate the beneficial effect of adding hexamethylene diamine (HMD) on the reaction temperature required for catalyst extraction. For Examples 31-33, Example 1 was repeated, but the mixing time was 20 minutes, and the temperature was varied as indicated in Table 6. For Examples 34-36, Example 5 was repeated, and the temperature was varied as indicated in Table 6.

**Table 6**

| **Effect of hexamethylene diamine on temperature for catalyst extraction.** | | | |
|---|---|---|---|
| Example | Temp (°C) | KLL | Zn/HMD |
| 31 | 65 | 16.76 | No HMD |
| 32 | 55 | 13.25 | No HMD |
| 33 | 45 | 8.06 | No HMD |
| 34 | 65 | 84.42 | 1.2 |
| 35 | 55 | 82.91 | 1.2 |
| 36 | 45 | 82.00 | 1.2 |

The data summarized in Table 6 represent evaluations of catalyst extraction performed at varying temperature from 45 to 65 degrees Celsius, with and without HMD present. Examples 31-33 show that catalyst extraction increases linearly with increasing temperature (represented by KLL). Examples 34-36 show that catalyst extraction does not require increased temperature when HMD is added.

### Examples 37-44

These Examples demonstrate the beneficial effect of adding hexamethylene diamine (HMD) on the mixing time required for catalyst extraction. For Examples 37-40, Example 31 was repeated, and the mixing time was varied as indicated in Table 7. For Examples 41-44, Example 5 was repeated, and the mixing time was varied as indicated in Table 7.

**Table 7**

| **Effect of hexamethylene diamine on mixing time required for catalyst extraction.** | | | |
|---|---|---|---|
| Example | Mixing Time | KLL | Zn/HMD |
| 37 | 20 | 16.13 | No HMD |
| 38 | 10 | 14.86 | No HMD |
| 39 | 5 | 14.49 | No HMD |
| 40 | 1 | 11.05 | No HMD |
| 41 | 10 | 84.42 | 1.2 |
| 42 | 5 | 114.34 | 1.2 |
| 43 | 1 | 98.24 | 1.2 |
| 44 | 0.5 | 56.23 | 1.2 |

The data summarized in Table 7 represent evaluations of catalyst extraction performed at varying mixing time from 20 minutes to 30 seconds, with and without HMD present. Examples 37-40 show that a decrease in catalyst extraction occurs when the mixing time is decreased to less than 5 minutes. Examples 41-44 show that catalyst extraction does not decrease until the mixing time is decreased to less than 1 minute, when HMD added.

### Examples 45-49

These Examples demonstrate the beneficial effect of adding hexamethylene diamine (HMD) and bis-hexamethylenetriamine to the mixing section of a mixer/settler, rather than to the feed line to this mixing section. Results are shown in Table 8.

**Table 8**

| **Effect of additive addition point.** | | | | | |
|---|---|---|---|---|---|
| Example | Addition Point | Additive | Mixing Time | KLL | Stable Emulsion |
| 45 | Mixer | HMD | 20 | 23 | No |
| 46 | Mixer | BHMT | 20 | 80 | No |
| 47 | Feed Line | HMD | N/A | 14 | Yes |
| 48 | Feed Line | BHMT | N/A | 14 | Yes |

Examples 45-48 show that addition of the additives HMD or BHMT directly to the mixer system of a catalyst extraction system causes a beneficial increase in catalyst recovery, as indicated by increased KLL.

### Examples 49-53

These Examples demonstrate the ability of complex of zinc chlioride (ZnCl₂) and bis-hexamethylenetriamine (BHMT) to catalyze the cyclization of adiponitrile (ADN) to 2-cyanocyclopentylideneimine (CPI) under conditions encountered when a raffinate stream is refined to produce purified ADN.

A simulated raffinate composition which was obtained from the tails stream of a column for removal of pentenenitriles from dinitriles (ie. column K'₂ and stream 630 in Figure 3) was used for the following examples. This raffinate had the following composition: 94% adiponitrile, 4% methylglutaronitrile, 0.1% pentenenitriles, 0.5% ethylsuccinonitrile, and 271 ppm zinc. To simulate conditions in a distillation column to distill dinitriles the raffinate was heated to 180°C.

Various additives were then added to the heated mixture. The composition of these additives is shown in Table 9.

**Table 9**

| **Amount of additive**. | | | |
|---|---|---|---|
| Example | Additive | Amount of BHMT | Zn/BHMT |
| 48 | BHMT + ZnCl₂ | 1 wt% | 1 |
| 49 | BHMT + ZnCl₂ | 2 wt% | 0.5 |
| 50 | BHMT + ZnCl₂ | 0.5 wt% | 2 |
| 51 | BHMT | 2 wt% | N/A |
| 52 | ZnCl₂ | 0 | N/A |

In Table 9, it will be understood that the amount of BHMT is based on the total weight of the raffinate composition before addition of the additive. It will be further understood that the ratio of Zn/BHMT is expressed in terms of equivalents of Zn per mole of BHMT. The amount of ZnCl₂ added as per Example 53 (EX 53) was 3 wt%, based on the total weight of the raffinate composition before addition of the ZnCl₂.

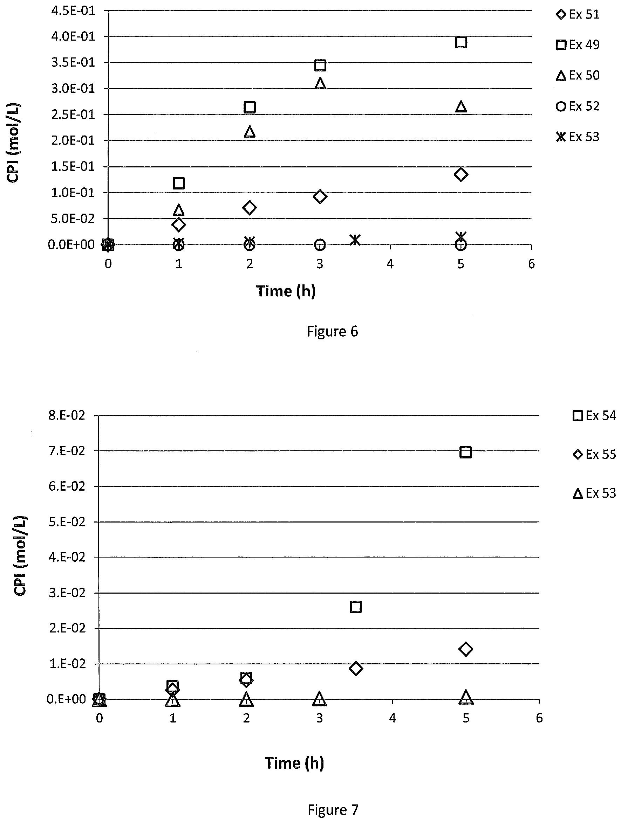

After the addition of the additive, samples of the mixture were taken at 1 hour, 2 hours, 3 hours and 5 hours. These samples were analyzed, and the concentration of CPI in the samples was determined in terms of CPI (mol/L), i.e. moles of CPI per liter of the mixture. Results are shown in Figure 6.

Figure 6 shows that CPI was not formed according to Example 51 (EX 51), wherein the additive included BHMT in the absence of ZnCl₂. Figure 6 also shows that CPI was not formed according to Example 52 (EX 52), wherein the additive included ZnCl₂ in the absence of BHMT. However, Figure 6 shows that considerable amounts of 2-cyanocyclopentylideneimine (CPI) were formed according to Examples 48-50 (EX 48 to EX 50) in increasing quantities over time when the additive included both BHMT and ZnCl₂.

### Examples 54-56

These Examples demonstrate the ability of a complex of zinc chlioride (ZnCl₂) and hexamethylenediamine (HMD) to catalyze the cyclization of adiponitrile (ADN) to 2-cyanocyclopentylideneimine (CPI) under conditions encountered when a raffinate stream is refined to produce purified ADN.

A raffinate material which was obtained from the tails stream of a column for removal of pentenenitriles from dinitriles (ie. column K'₂ and stream 630 in Figure 3) was used for the following examples. This raffinate had the following composition: 94% adiponitrile, 4% methylglutaronitrile, 0.1% pentenenitriles, 0.5% ethylsuccinonitrile, and 271 ppm zinc. To simulate conditions in a distillation column to distill dinitriles the raffinate was heated to 180°C.

Various additives were then added to the heated mixture. The composition of these additives is shown in Table 10.

**Table 10**

| **Amount of additive.** | | | |
|---|---|---|---|
| Example | Additive | Amount of HMD | Zn/HMD |
| 54 | HMD + ZnCl₂ | 0.5 wt% | 1 |
| 55 | ZnCl₂ | 0 | N/A |
| 56 | HMD | 0.5 wt% | N/A |

In Table 10 it will be understood that the amount of HMD is based on the total weight of the raffinate composition before addition of the additive. It will be further understood that the ratio of Zn/HMD is expressed in terms of equivalents of Zn per mole of HMD. The amount of ZnCl₂ added as per Example 55 (EX 55) was 0.6 wt%, based on the total weight of the raffinate composition before addition of the ZnCl₂.

After the addition of the additive, samples of the mixture were taken at various times including 1 hour, 2 hours, 3 hours, 3.5 hours and 5 hours. These samples were analyzed, and the concentration of CPI in the samples was determined in terms of CPI (mol/L), i.e. moles of CPI per liter of the mixture. Results are shown in Figure 7.

Figure 7 shows that CPI formation was negligible according to Example 55 (EX 55), wherein the additive included HMD in the absence of ZnCl₂. Figure 7 also shows that only small amounts of CPI were formed according to Example 54 (EX 54), wherein the additive included ZnCl₂ in the absence of HMD. However, Figure 6 shows that considerable amounts of 2-cyanocyclopentylideneimine (CPI) were formed according to Example 53 (EX 53) in increasing quantities over time when the additive included both HMD and ZnCl₂, especially when the mixture was heated for 3.5 and 5 hours.

## Claims

1. A process for recovering diphosphonite-containing compounds from a feed mixture comprising diphosphonite-containing compounds, organic mononitriles, organic dinitriles and a Lewis acid in a multistage countercurrent liquid-liquid extractor with extraction solvent comprising aliphatic hydrocarbon, cycloaliphatic hydrocarbon or a mixture of aliphatic and cycloaliphatic hydrocarbon, said process comprising:
a) flowing the feed mixture to the first stage of the multistage countercurrent liquid-liquid extractor; and
b) contacting the feed mixture with extraction solvent from the light phase of the second stage of the multistage countercurrent liquid-liquid extractor,
wherein the first stage of the multistage countercurrent liquid-liquid extractor comprises a mixing section and a settling section, wherein the mixing section provides a mixed phase comprising a light phase and a heavy phase, wherein a light phase separates from a heavy phase in the settling section, wherein a Lewis base is added to the mixing section of the first stage of the multistage countercurrent liquid-liquid extractor, wherein the light phase comprises extraction solvent and extracted diphosphonite-containing compounds, wherein the heavy phase comprises organic mononitriles, organic dinitriles and a complex of said Lewis acid and said Lewis base, wherein at least a portion of the light phase is withdrawn from the settling section and treated to recover diphosphonite-containing compounds extracted into the light phase, wherein at least a portion of the heavy phase is passed to the second stage of the multistage countercurrent liquid-liquid extractor;
wherein the Lewis base is selected from the group consisting of:
a) anhydrous ammonia, pyridine, alkylamine, dialkylamine, trialkylamine wherein the alkyl groups have 1 to 12 carbon atoms; and
b) polyamine.

2. The process of claim 1, wherein the Lewis base is cofed to the mixing section along with the feed mixture comprising diphosphonite-containing compounds, organic mononitriles, organic dinitriles and Lewis acid.

3. The process of claim 1, wherein the Lewis base is separately fed to the mixing section apart from the feed mixture comprising diphosphonite-containing compounds, organic mononitriles, organic dinitriles and Lewis acid and the extraction solvent feed.

4. The process of claim 1, wherein the Lewis acid is ZnCl₂.

5. The process of claim 1, wherein the extraction solvent feed from the second stage comprises at least 1000 ppm of diphosphonite-containing compounds.

6. The process of claim 1, wherein the diphosphonite-containing compound is:
(R¹)(R²-O)P-O-Y-O-P(O-R³)(R⁴) I
where R¹ and R² are each independently identical or different, separate or bridged organic radicals; R³ and R⁴ are each independently identical or different, separate or bridged organic radicals; and Y is a bridging group.

7. The process of claim 1, wherein the diphosphonite-containing compound is selected from the group consisting of: 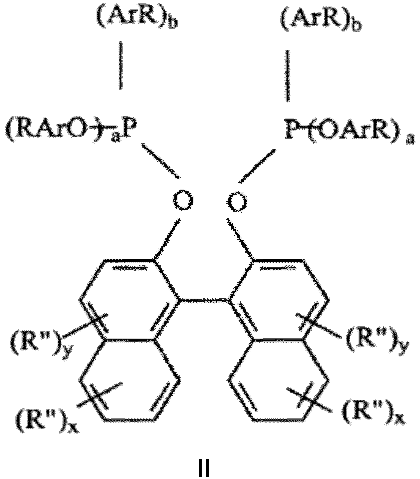 and 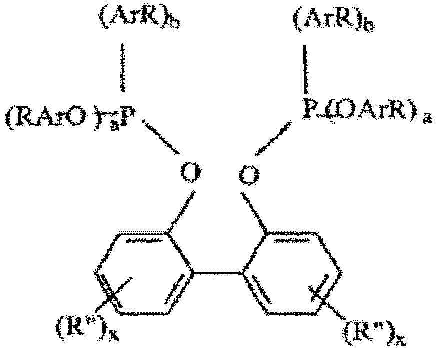 wherein:
x=0 to 4;
y=0 to 2;
a is 1 and b is 1;
each Ar is individually phenyl or naphthyl, and the two Ar groups that are directly or indirectly (through an oxygen) bonded to the same phosphorus atom may be linked to each other by a linking unit selected from the group consisting of direct bond, alkylidene, secondary or tertiary amine, oxygen, sulfide, sulfone, and sulfoxide;
each R is individually hydrogen, ethenyl, propenyl, acryloyl, methacryloyl, an organic radical with a terminal ethenyl, propenyl, acryloyl, or methacryloyl group, linear or branched alkyl, cycloalkyl, acetal, ketal, aryl, alkoxy, cycloalkoxy, aryloxy, formyl, ester, fluorine, chlorine, bromine, perhaloalkyl, hydrocarbylsulfinyl, hydrocarbylsulfonyl, hydrocarbylcarbonyl or cyclic ether;
each Ar can be further substituted with linear or branched alkyl, cycloalkyl, acetal, ketal, aryl, alkoxy, cycloalkoxy, aryloxy, formyl, ester, fluorine, chlorine, bromine, perhaloalkyl, hydrocarbylsulfinyl, hydrocarbylsulfonyl, hydrocarbylcarbonyl or cyclic ether;
each R" is individually hydrogen, ethenyl, propenyl, an organic radical with a terminal ethenyl or propenyl group, linear or branched alkyl, cycloalkyl, acetal, ketal, aryl, alkoxy, cycloalkoxy, aryloxy, formyl, ester, fluorine, chlorine, bromine, perhaloalkyl, hydrocarbylsulfinyl, hydrocarbylsulfonyl, hydrocarbylcarbonyl or cyclic ether.

8. The process of claim 7, wherein at least one R represents ethenyl, propenyl, acryloyl, methacryloyl or the organic radical with a terminal ethenyl, propenyl, acryloyl, or methacryloyl group or at least one R" represents ethenyl, propenyl, or the organic radical with a terminal ethenyl or propenyl group.

9. The process of claim 1, wherein the diphosphonite-containing compound is:

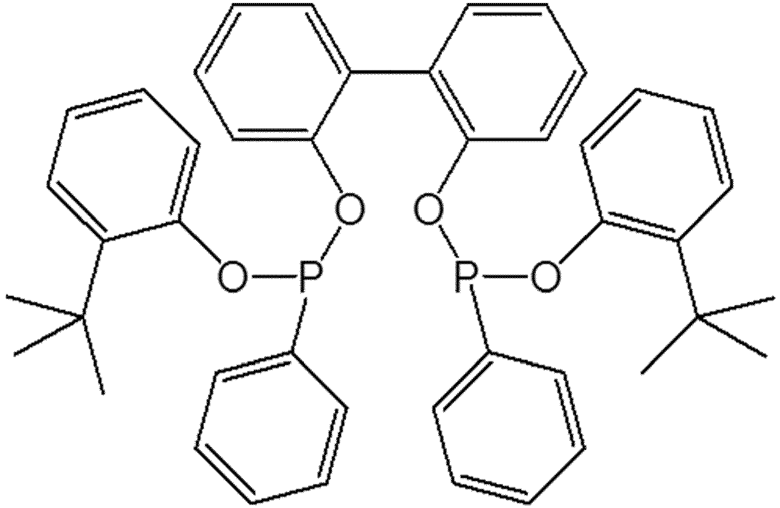

10. The process of claims 4, wherein at least a portion of the diphosphonite-containing compound is complexed with zero valent Ni.

11. The process of one of claims 1, 2 or 3 wherein at least one stage of the extraction is carried out above 40°C.

12. The process of claim 1 wherein the Lewis base is a monodentate triarylphosphite wherein the aryl groups are unsubstituted or substituted with alkyl groups having 1 to 12 carbon atoms, and wherein the aryl groups may be interconnected.

13. The process of any one of claims 1, 2 or 3 wherein the extraction solvent is cyclohexane.

14. The process of claim 1 wherein the feed mixture is an effluent stream from a hydrocyanation process; and optionally,
wherein the hydrocyanation process includes a 3-pentenenitrile hydrocyanation process; and optionally,
wherein the hydrocyanation process includes a 1,3-butadiene hydrocyanation process.

15. The process of claim 1, wherein the first stage of the multistage countercurrent liquid-liquid extractor takes place in an extraction column, wherein the entire column is a settling section comprising a mixing section between a heavy phase collection section and a light phase collection section, and wherein the Lewis base is added to the mixing section; or
wherein the first stage of the multistage countercurrent liquid-liquid extractor takes place in an mixer-settler, wherein the mixer-settler comprises a settling section which is separate from the mixing section, and wherein the Lewis base is added to the mixing section.

## Patentansprüche

1. Verfahren zum Gewinnen von diphosphonithaltigen Verbindungen aus einem Einsatzgemisch, das diphosphonithaltige Verbindungen, organische Mononitrile, organische Dinitrile und eine Lewis-Säure umfasst, in einem mehrstufigen Gegenstrom-Flüssig-Flüssig-Extraktor mit einem Extraktionslösungsmittel, das aliphatischen Kohlenwasserstoff, cycloaliphatischen Kohlenwasserstoff oder ein Gemisch von aliphatischem und cycloaliphatischem Kohlenwasserstoff umfasst, wobei das Verfahren umfasst:
a) Leiten des Einsatzgemischs in die erste Stufe des mehrstufigen Gegenstrom-Flüssig-Flüssig-Extraktors; und
b) Inkontaktbringen des Einsatzgemischs mit Extraktionslösungsmittel aus der leichten Phase der zweiten Stufe des mehrstufigen Gegenstrom-Flüssig-Flüssig-Extraktors,
wobei die erste Stufe des mehrstufigen Gegenstrom-Flüssig-Flüssig-Extraktors einen Mischabschnitt und einen Absetzabschnitt umfasst, wobei der Mischabschnitt eine gemischte Phase bereitstellt, die eine leichte Phase und eine schwere Phase umfasst, wobei sich in dem Absetzabschnitt eine leichte Phase von einer schweren Phase trennt, wobei dem Mischabschnitt der ersten Stufe des mehrstufigen Gegenstrom-Flüssig-Flüssig-Extraktors eine Lewis-Base zugesetzt wird, wobei die leichte Phase Extraktionslösungsmittel und extrahierte diphosphonithaltige Verbindungen umfasst, wobei die schwere Phase organische Mononitrile, organische Dinitrile und einen Komplex der Lewis-Säure und der Lewis-Base umfasst, wobei wenigstens ein Teil der leichten Phase aus dem Absetzabschnitt entnommen wird und behandelt wird, um in die leichte Phase extrahierte diphosphonithaltige Verbindungen zu gewinnen, wobei wenigstens ein Teil der schweren Phase an die zweite Stufe des mehrstufigen Gegenstrom-Flüssig-Flüssig-Extraktors weitergeleitet wird;
wobei die Lewis-Base aus der Gruppe bestehend aus:
a) wasserfreiem Ammoniak, Pyridin, Alkylamin, Dialkylamin, Trialkylamin, wobei die Alkylgruppen 1 bis 12 Kohlenstoffatome aufweisen; und
b) Polyamin
ausgewählt wird.

2. Verfahren nach Anspruch 1, wobei die Lewis-Base dem Mischabschnitt zusammen mit dem Einsatzgemisch, das diphosphonithaltige Verbindungen, organische Mononitrile, organische Dinitrile und Lewis-Säure umfasst, zugeführt wird.

3. Verfahren nach Anspruch 1, wobei die Lewis-Base dem Mischabschnitt getrennt von dem Einsatzgemisch, das diphosphonithaltige Verbindungen, organische Mononitrile, organische Dinitrile und Lewis-Säure umfasst, und der Extraktionslösungsmittelzufuhr zugeführt wird.

4. Verfahren nach Anspruch 1, wobei sich bei der Lewis-Säure um ZnCl₂ handelt.

5. Verfahren nach Anspruch 1, wobei die Extraktionslösungsmittelzufuhr aus der zweiten Stufe mindestens 1000 ppm diphosphonithaltige Verbindungen umfasst.

6. Verfahren nach Anspruch 1, wobei es sich bei der diphosphonithaltigen Verbindung um
(R¹)(R²-O)P-O-Y-O-P(O-R³)(R⁴) I
handelt, wobei R¹ und R² jeweils unabhängig für gleiche oder verschiedene, separate oder verbrückte organische Reste stehen; R³ und R⁴ jeweils unabhängig für gleiche oder verschiedene, separate oder verbrückte organische Reste stehen und Y für eine Brückengruppe steht.

7. Verfahren nach Anspruch 1, wobei die diphosphonithaltige Verbindung aus der Gruppe bestehend aus: 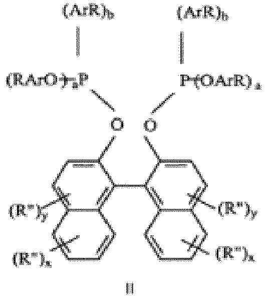 und 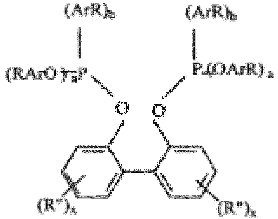 ausgewählt wird, wobei:
x = 0 bis 4;
y = 0 bis 2;
a für 1 steht und b für 1 steht;
Ar jeweils individuell für Phenyl oder Naphthyl steht und die beiden Ar-Gruppen, die direkt oder indirekt (über einen Sauerstoff) an dasselbe Phosphoratom gebunden sind, über eine Verknüpfungseinheit aus der Gruppe bestehend aus direkter Bindung, Alkyliden, sekundärem oder tertiärem Amin, Sauerstoff, Sulfid, Sulfon und Sulfoxid miteinander verknüpft sein können;
R jeweils individuell für Wasserstoff, Ethenyl, Propenyl, Acryloyl, Methacryloyl, einen organischen Rest mit einer endständigen Ethenyl-, Propenyl-, Acryloyl- oder Methacryloylgruppe, lineares oder verzweigtes Alkyl, Cycloalkyl, Acetal, Ketal, Aryl, Alkoxy, Cycloalkoxy, Aryloxy, Formyl, Ester, Fluor, Chlor, Brom, Halogenalkyl, Hydrocarbylsulfinyl, Hydrocarbylsulfonyl, Hydrocarbylcarbonyl oder cyclischen Ether steht;
Ar jeweils weiter durch lineares oder verzweigtes Alkyl, Cycloalkyl, Acetal, Ketal, Aryl, Alkoxy, Cycloalkoxy, Aryloxy, Formyl, Ester, Fluor, Chlor, Brom, Halogenalkyl, Hydrocarbylsulfinyl, Hydrocarbylsulfonyl, Hydrocarbylcarbonyl oder cyclischen Ether substituiert sein kann;
R" jeweils individuell für Wasserstoff, Ethenyl, Propenyl, einen organischen Rest mit einer endständigen Ethenyl- oder Propenylgruppe, lineares oder verzweigtes Alkyl, Cycloalkyl, Acetal, Ketal, Aryl, Alkoxy, Cycloalkoxy, Aryloxy, Formyl, Ester, Fluor, Chlor, Brom, Halogenalkyl, Hydrocarbylsulfinyl, Hydrocarbylsulfonyl, Hydrocarbylcarbonyl oder cyclischen Ether steht.

8. Verfahren nach Anspruch 7, wobei mindestens ein R für Ethenyl, Propenyl, Acryloyl, Methacryloyl oder den organischen Rest mit einer endständigen Ethenyl-, Propenyl-, Acryloyl- oder Methacryloylgruppe steht oder mindestens ein R" für Ethenyl, Propenyl oder den organischen Rest mit einer endständigen Ethenyl- oder Propenylgruppe steht.

9. Verfahren nach Anspruch 1, wobei sich bei der diphosphonithaltigen Verbindung um 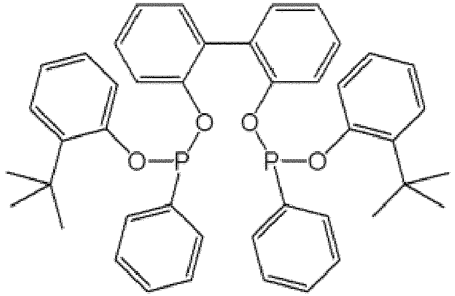 handelt.

10. Verfahren nach Anspruch 4, bei dem wenigstens ein Teil der diphosphonithaltigen Verbindung mit nullwertigem Ni komplexiert ist.

11. Verfahren nach einem der Ansprüche 1, 2 oder 3, wobei wenigstens eine Stufe der Extraktion bei über 40 °C durchgeführt wird.

12. Verfahren nach Anspruch 1, wobei es sich bei der Lewis-Base um ein monodentates Triarylphosphit handelt, wobei die Arylgruppen unsubstituiert sind oder durch Alkylgruppen mit 1 bis 12 Kohlenstoffatomen substituiert sind und wobei die Arylgruppen miteinander verbunden sein können.

13. Verfahren nach einem der Ansprüche 1, 2 oder 3, wobei es sich bei dem Extraktionslösungsmittel um Cyclohexan handelt.

14. Verfahren nach Anspruch 1, wobei sich bei dem Einsatzgemisch um einen Austragsstrom aus einem Hydrocyasnierungsverfahren handelt; und gegebenenfalls
wobei das Hydrocyanierungsverfahren ein 3-Pentennitril-Hydrocyanierungsverfahren umfasst und gegebenenfalls
wobei das Hydrocyanierungsverfahren ein 1,3-Butadien-Hydrocyanierungsverfahren umfasst.

15. Verfahren nach Anspruch 1, wobei die erste Stufe des mehrstufigen Gegenstrom-Flüssig-Flüssig-Extraktors in einer Extraktionssäule stattfindet, wobei die gesamte Säule einen Absetzabschnitt darstellt, der einen Mischabschnitt zwischen einem Abschnitt zum Sammeln der schweren Phase und einem Abschnitt zum Sammeln der leichten Phase umfasst, und wobei Lewis-Base in den Mischabschnitt zurückgeführt wird; oder
wobei die erste Stufe des mehrstufigen Gegenstrom-Flüssig-Flüssig-Extraktors in einem Mischer-Absetzer stattfindet, wobei der Mischer-Absetzer einen Absetzabschnitt umfasst, der vom Mischabschnitt getrennt ist, und wobei die Lewis-Base dem Mischabschnitt zugesetzt wird.

## Revendications

1. Procédé de récupération de composés contenant un diphosphonite à partir d'un mélange d'alimentation comprenant des composés contenant un diphosphonite, des mononitriles organiques, des dinitriles organiques et a acide de Lewis dans un extracteur liquide-liquide à contre-courant à étages multiples avec i, solvant d'extraction comprenant un hydrocarbure aliphatique, un hydrocarbure cycloaliphatique ou un mélange d'hydrocarbures aliphatique et cycloaliphatique, ledit procédé comprenant :
a) l'écoulement du mélange d'alimentation vers le premier étage de l'extracteur liquide-liquide à contre-courant à étages multiples ; et
b) la mise en contact du mélange d'alimentation avec le solvant d'extraction provenant de la phase du deuxième étage de l'extracteur liquide-liquide à contre-courant à étages multiples,
dans lequel le premier étage de l'extracteur liquide-liquide à contre-courant à étages multiples comprend une section de mélange et une section de décantation, dans lequel la section de mélange produit une phase mixte comprenant une phase légère et une phase lourde, dans lequel une phase légère se sépare d'une phase lourde dans la section de décantation, dans lequel une base de Lewis est ajoutée à la section de mélange du premier étage de l'extracteur liquide-liquide à contre-courant à étages multiples, dans lequel la phase légère comprend un solvant d'extraction et les composés contenant un diphosphonite extraits, dans lequel la phase lourde comprend des mononitriles organiques, des dinitriles organiques et un complexe dudit acide de Lewis et de ladite base de Lewis, dans lequel au moins une partie de la phase légère est retirée de la section de décantation et traitée pour récupérer les composés contenant un diphosphonite extraits dans la phase légère, dans lequel au moins une partie de la phase lourde est transférée vers le deuxième étage de l'extracteur liquide-liquide à contre-courant à étages multiples ;
dans lequel la base de Lewis est choisie dans le groupe constitué de :
a) l'ammoniac, la pyridine, une alkylamine, une dialkylamine, une trialkylamine anhydres où les groupes alkyle comportent 1 à 12 atomes de carbone ; et
b) une polyamine.

2. Procédé de la revendication 1, dans lequel la base de Lewis est co-alimentée dans la section de mélange avec le mélange d'alimentation comprenant des composés contenant un diphosphonite, des mononitriles organiques, des dinitriles organiques et un acide de Lewis.

3. Procédé de la revendication 1, dans lequel la base de Lewis est alimentée dans la section de mélange séparément du mélange d'alimentation comprenant des composés contenant un diphosphonite, des mononitriles organiques, des dinitriles organiques et un acide de Lewis et la charge de solvant d'extraction.

4. Procédé de la revendication 1, dans lequel l'acide de Lewis est ZnCl₂.

5. Procédé de la revendication 1, dans lequel le solvant d'extraction alimenté depuis le deuxième étage comprend au moins 1000 ppm de composés contenant un diphosphonite.

6. Procédé de la revendication 1, dans lequel le composé contenant un diphosphonite est :
(R¹)(R²-^{O})P-O-Y-O-P(O-R³)(R⁴) I
où R¹ et R² sont chacun indépendamment des radicaux organiques identiques ou différents, séparés ou pontés ; R³ et R⁴ sont chacun indépendamment des radicaux organiques identiques ou différents, séparés ou pontés ; et Y est un groupe de pontage.

7. Procédé de la revendication 1, dans lequel le composé contenant un diphosphonite est choisi dans le groupe constitué de : 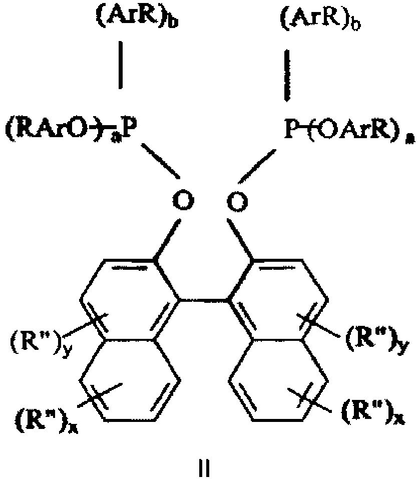 et 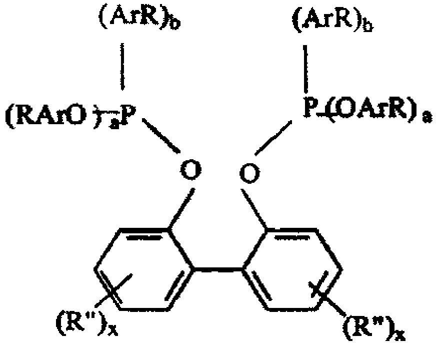 dans lequel :
x = 0 à 4 ;
y = 0 à 2 ;
a est 1 et b est 1 ;
chaque Ar est individuellement phényle ou naphtyle, et les deux groupes Ar qui sont directement ou indirectement (par l'intermédiaire d'un oxygène) liés au même atome de phosphore peuvent être liés l'un à l'autre par une unité de liaison choisie dans le groupe constitué d'une liaison directe, alkylidène, une amine secondaire ou tertiaire, oxygène, sulfure, sulfone et sulfoxyde ;
chaque R est individuellement hydrogène, éthényle, propényle, acryloyle, méthacryloyle, un radical organique avec un groupe éthényle, propényle, acryloyle ou méthacryloyle terminal, alkyle linéaire ou ramifié, cycloalkyle, acétal, cétal, aryle, alcoxy, cycloalcoxy, aryloxy, formyle, ester, fluor, chlore, brome, perhalogénoalkyle, hydrocarbylsulfinyle, hydrocarbylsulfonyle, hydrocarbylcarbonyle ou éther cyclique ; chaque Ar peut en outre être substitué par alkyle linéaire ou ramifié, cycloalkyle, acétal, cétal, aryle, alcoxy, cycloalcoxy, aryloxy, formyle, ester, fluor, chlore, brome, perhalogénoalkyle, hydrocarbylsulfinyle, hydrocarbylsulfonyle, hydrocarbylcarbonyle ou éther cyclique ;
chaque R" est individuellement hydrogène, éthényle, propényle, un radical organique avec un groupe éthényle ou propényle terminal, alkyle linéaire ou ramifié, cycloalkyle, acétal, cétal, aryle, alcoxy, cycloalcoxy, aryloxy, formyle, ester, fluor, chlore, brome, perhalogénoalkyle, hydrocarbylsulfinyle, hydrocarbylsulfonyle, hydrocarbylcarbonyle ou éther cyclique.

8. Procédé de la revendication 7, dans lequel au moins un R représente éthényle, propényle, acryloyle, méthacryloyle ou le radical organique avec un groupe éthényle, propényle, acryloyle ou méthacryloyle terminal ou au moins un R" représente éthényle, propényle, ou le radical organique avec un groupe éthényle ou propényle terminal.

9. Procédé de la revendication 1, dans lequel le composé contenant un diphosphonite est :

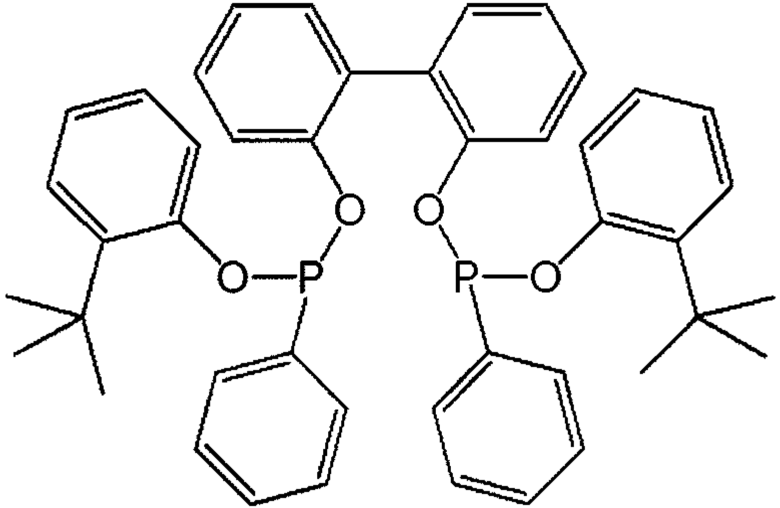

10. Procédé des revendications 4, dans lequel au moins une partie du composé contenant un diphosphonite est complexé avec Ni zéro-valent.

11. Procédé de l'une des revendications 1, 2 ou 3 dans lequel au moins une étape de l'extraction est conduite au-dessus de 40 °C.

12. Procédé de la revendication 1 dans lequel la base de Lewis est un triarylphosphite monodentate dans lequel les groupes aryle sont non substitués ou substitués par des groupes alkyle ayant 1 à 12 atomes de carbone, et dans lequel les groupes aryle peuvent être interconnectés.

13. Procédé de l'une quelconque des revendications 1, 2 ou 3 dans lequel le solvant d'extraction est cyclohexane.

14. Procédé de la revendication 1 dans lequel le mélange d'alimentation est un flux d'effluent provenant d'un procédé d'hydrocyanation ; et facultativement, dans lequel le procédé d'hydrocyanation comprend un procédé d'hydrocyanation de 3-pentènenitrile ; et facultativement,
dans lequel le procédé d'hydrocyanation comprend un procédé d'hydrocyanation de 1,3-butadiène.

15. Procédé de la revendication 1, dans lequel la première étape de l'extracteur liquide-liquide à contre-courant à étages multiples est conduite dans une colonne d'extraction, dans lequel la colonne entière est une section de décantation comprenant une section de mélange entre une section de collecte de phase lourde et une section de collecte de phase légère, et dans lequel la base de Lewis est ajoutée à la section de mélange ; ou dans lequel la première étape de l'extracteur liquide-liquide à contre-courant à étages multiples est conduite dans un mélangeur-décanteur, dans lequel le mélangeur-décanteur comprend une section de décantation qui est séparée de la section de mélange, et dans lequel la base de Lewis est ajoutée à la section de mélange.
